# EUROPEAN PATENT APPLICATION

(11) **EP 4 226 791 A1**
(43) Date of publication of application: **16.08.2023**
(21) Application number: 21931521.5
(22) Date of filing: 17.03.2021
(51) Int. Cl.: A24F 40/50, A24F 40/465

(54) **SUCTION DEVICE, PROGRAM, AND SYSTEM**

(71) Applicant: Japan Tobacco Inc., Tokyo 105-6927 (JP)
(72) Inventor: KAWASAKI, Reijiro, Tokyo 130-8603 (JP); SERITA, Kazutoshi, Tokyo 130-8603 (JP)
(74) Representative: Hoffmann Eitle
(86) International application number: PCT/JP2021/010849
(87) International publication number: WO 2022/195769

(57) **Abstract**

[Problem] To provide, in an induction heating-type suction device, a mechanism that is capable of more appropriately determining the presence of a susceptor-containing substrate. [Solution] Provided is a suction device including: a power supply unit that supplies power; a holding part that has an internal space and an opening, which allows the internal space to communicate with the exterior, and that holds a substrate that is inserted into the internal space from the opening and that contains a susceptor, which generates heat when a variable magnetic field penetrates thereinto, and an aerosol source; an electromagnetic induction source that uses the power supplied from the power supply unit to generate the variable magnetic field in the internal space of the holding part; a detection unit that detects information about a partial space, which is a portion of the internal space on the opening side; and a control unit that determines whether the substrate is held in the holding part on the basis of the partial space information detected by the detection unit.

## Description

### Technical Field

The present invention relates to an inhaler device, a program, and a system.

### Background Art

Inhaler devices, such as e-cigarettes and nebulizers, for generating a substance to be inhaled by users are widespread. For example, the inhaler devices generate an aerosol having a flavor component imparted thereto, by using a substrate including an aerosol source for generating the aerosol, a flavor source for imparting the flavor component to the generated aerosol, and the like. Users can enjoy the flavor by inhaling the aerosol having the flavor component imparted thereto, which is generated by the inhaler devices. An action of a user inhaling an aerosol is hereinafter referred to as a puff or a puff action.

Inhaler devices using an external heat source such as a heating blade had been dominant until recently. However, in recent years, techniques related to inhaler devices of induction heating type have been actively developed. For example, Patent Literature 1 below discloses a technique of determining whether a substrate including a susceptor is inserted in an inhaler device, based on an impulse response of an induction coil when induction heating of the susceptor is attempted by generating a varying magnetic field from the induction coil.

### Citation List

### Patent Literature

Patent Literature 1: WO 2020/260884 A1

### Summary of Invention

### Technical Problem

In the technique described in Patent Literature 1 above, the varying magnetic field is generated from the induction coil in the process of determining whether the substrate including the susceptor is present. Thus, when the susceptor is not present, a large leakage flux may occur.

Accordingly, the present invention has been made in view of the issue described above, and it is an object of the present invention to provide a mechanism capable of appropriately determining whether a substrate including a susceptor is present in an inhaler device of induction heating type.

### Solution to Problem

To overcome the issue described above, an aspect of the present invention provides an inhaler device including: a power supply configured to supply electric power; a holder having an internal space and an opening that allows the internal space to communicate with outside and configured to hold a substrate inserted into the internal space through the opening, the substrate including an aerosol source and a susceptor configured to produce heat upon being penetrated by a varying magnetic field; an electromagnetic induction source configured to generate the varying magnetic field in the internal space of the holder by using the electric power supplied from the power supply; a detector configured to detect information of a partial space that is a portion of the internal space adjacent to the opening; and a controller configured to determine whether the substrate is held by the holder, based on the information of the partial space detected by the detector.

The controller may be configured to determine whether the substrate including the susceptor is held by the holder, based on a time-series change in the information of the partial space.

The controller may be configured to: determine which of a first state, a second state, or a third state a state of the partial space is, based on the information of the partial space detected by the detector, the first state being a state in which an object is not present in the partial space, the second state being a state in which an object including a conductor is present in the partial space, the third state being a state in which an object not including a conductor is present in the partial space; and determine whether the substrate including the susceptor is held by the holder, based on a time-series change in the state of the partial space.

The controller may be configured to determine that the substrate including the susceptor is held by the holder in a case where the state of the partial space changes from the first state to the second state and then to the third state.

The controller may be configured to determine that the substrate including the susceptor that has been held by the holder is removed in a case where the state of the partial space changes from the third state to the second state and then to the first state.

The controller may be configured to determine that the substrate including the susceptor is not held by the holder in a case where the state of the partial space does not change from the first state.

The controller may be configured to determine that the substrate including the susceptor is inserted halfway and then removed in a case where the state of the partial space changes from the first state to the second state and then to the first state.

The controller may be configured to identify the substrate held by the holder, based on the time-series change in the information of the partial space.

The controller may be configured to start induction heating using the electromagnetic induction source in response to determining that the substrate including the susceptor is held by the holder.

The substrate may include a first portion where the susceptor is distributed and a second portion where the susceptor is not distributed, and the second portion may be located in the partial space when the substrate is held by the holder.

The electromagnetic induction source may be disposed outside the internal space, and the detector may be disposed outside the internal space to be closer to the opening than the electromagnetic induction source is.

The detector may be a non-contact proximity sensor.

The detector may be a capacitive proximity sensor.

The detector may be an inductive proximity sensor.

To overcome the issue described above, another aspect of the present invention provides a program to be executed by a computer that controls an inhaler device, the inhaler device including: a power supply configured to supply electric power; a holder having an internal space and an opening that allows the internal space to communicate with outside and configured to hold a substrate inserted into the internal space through the opening, the substrate including an aerosol source and a susceptor configured to produce heat upon being penetrated by a varying magnetic field; an electromagnetic induction source configured to generate the varying magnetic field in the internal space of the holder by using the electric power supplied from the power supply; and a detector configured to detect information of a partial space that is a portion of the internal space adjacent to the opening, the program causing determining whether the substrate is held by the holder, based on the information of the partial space detected by the detector to be performed.

To overcome the issue described above, another aspect of the present invention provides a system including: an inhaler device; and a substrate, the substrate including an aerosol source and a susceptor configured to produce heat upon being penetrated by a varying magnetic field, the inhaler device including: a power supply configured to supply electric power; a holder having an internal space and an opening that allows the internal space to communicate with outside and configured to hold the substrate inserted into the internal space through the opening; an electromagnetic induction source configured to generate the varying magnetic field in the internal space of the holder by using the electric power supplied from the power supply; a detector configured to detect information of a partial space that is a portion of the internal space adjacent to the opening; and a controller configured to determine whether the substrate is held by the holder, based on the information of the partial space detected by the detector.

### Advantageous Effects of Invention

As described above, the present invention provides a mechanism capable of appropriately determining whether a substrate including a susceptor is present in an inhaler device of induction heating type.

### Brief Description of Drawings

[Fig. 1] Fig. 1 is a schematic diagram of an inhaler device according to a configuration example.
[Fig. 2] Fig. 2 is a diagram schematically illustrating a configuration around a holder of the inhaler device according to the present embodiment and a configuration of a stick substrate.
[Fig. 3] Fig. 3 is a diagram schematically illustrating a positional relationship between the holder and the stick substrate in a first state.
[Fig. 4] Fig. 4 is a diagram schematically illustrating a positional relationship between the holder and the stick substrate in a second state.
[Fig. 5] Fig. 5 is a diagram schematically illustrating a positional relationship between the holder and the stick substrate in a third state.
[Fig. 6] Fig. 6 is a flowchart illustrating an example of a procedure of a process of determining whether a stick substrate is present, performed by the inhaler device according to the present embodiment.

### Description of Embodiments

A preferred embodiment of the present invention will be described in detail below with reference to the accompanying drawings. In the specification and the drawings, structural elements having substantially the same functional configuration are denoted by the same reference numerals, and redundant description thereof will be omitted.

### <1. Configuration example of inhaler device>

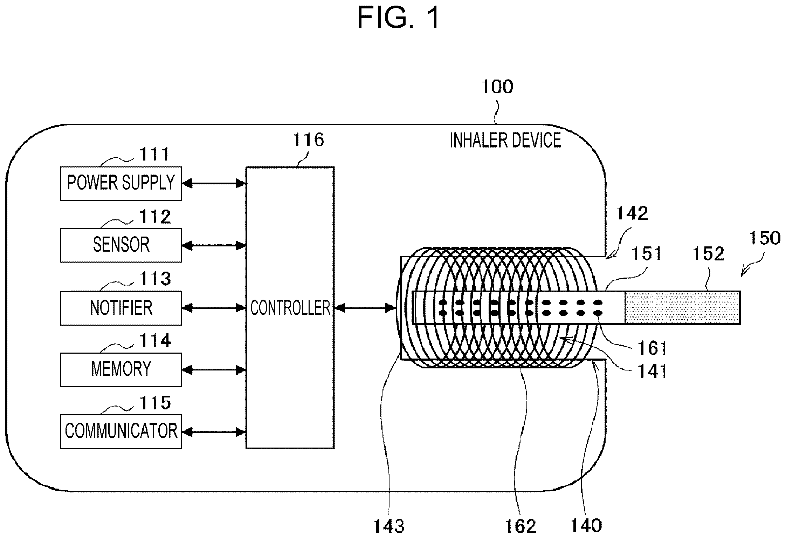

An inhaler device according to the present configuration example heats a substrate including an aerosol source by induction heating (IH) to generate an aerosol. The present configuration example will be described below with reference to Fig. 1.

Fig. 1 is a schematic diagram of the inhaler device according to the configuration example. As illustrated in Fig. 1, an inhaler device 100 according to the present configuration example includes a power supply 111, a sensor 112, a notifier 113, a memory 114, a communicator 115, a controller 116, a susceptor 161, an electromagnetic induction source 162, and a holder 140. A user performs inhalation while a stick substrate 150 is held by the holder 140. Each structural element will be sequentially described below.

The power supply 111 stores electric power. The power supply 111 supplies electric power to each structural element of the inhaler device 100. The power supply 111 may be, for example, a rechargeable battery such as a lithium ion secondary battery. The power supply 111 may be charged by being connected to an external power supply through a Universal Serial Bus (USB) cable or the like. In addition, the power supply 111 may be charged, by using a wireless power transmission technology, without being connected to a power-transmitting device. Further, the power supply 111 alone may be removed from the inhaler device 100 and replaced with a new power supply 111.

The sensor 112 detects various items of information regarding the inhaler device 100. The sensor 112 outputs the detected items of information to the controller 116. In an example, the sensor 112 may be a pressure sensor such as a condenser microphone, a flow sensor, or a temperature sensor. In response to detecting a numerical value in accordance with inhalation performed by a user, the sensor 112 outputs information indicating that the user has performed the inhalation to the controller 116. In another example, the sensor 112 may be an input device that accepts information input by the user, such as a button or a switch. In particular, the sensor 112 may include a button for inputting an instruction to start/stop generation of an aerosol. The sensor 112 outputs the information input by the user to the controller 116. In another example, the sensor 112 may be a temperature sensor that detects a temperature of the susceptor 161. The temperature sensor detects the temperature of the susceptor 161 based on, for example, an electrical resistance value of the electromagnetic induction source 162. The sensor 112 may detect the temperature of the stick substrate 150 held by the holder 140, based on the temperature of the susceptor 161.

The notifier 113 notifies the user of information. In an example, the notifier 113 may be a light-emitting device such as a light-emitting diode (LED). In this case, the notifier 113 emits different patterns of light when the power supply 111 needs to be charged, when the power supply 111 is being charged, when the inhaler device 100 has an anomaly, and so on. The pattern of light is a concept including a color, turn-on/turn-off timings, and so on. The notifier 113 may be, along with or instead of the light-emitting device, a display device that displays an image, a sound output device that outputs sound, or a vibration device that vibrates. In addition, the notifier 113 may notify the user of information indicating that the user can perform inhalation. The user is notified of the information indicating that the user can perform inhalation, in response to the temperature of the stick substrate 150 that produces heat by electromagnetic induction reaching a predetermined temperature.

The memory 114 stores various items of information for operation of the inhaler device 100. The memory 114 may be a non-volatile storage medium such as a flash memory. An example of the items of information stored in the memory 114 is items of information related to an operating system (OS) of the inhaler device 100, such as details of control performed on the various structural elements by the controller 116. Another example of the items of information stored in the memory 114 is items of information related to inhalation performed by the user, such as the number of times of inhalation, an inhalation time, and an accumulated inhalation time period.

The communicator 115 is a communication interface for transmitting and receiving information between the inhaler device 100 and another device. The communicator 115 performs communication in conformity with any wired or wireless communication standard. Such a communication standard may be, for example, a wireless local area network (LAN), a wired LAN, Wi-Fi (registered trademark), or Bluetooth (registered trademark). In an example, the communicator 115 transmits the items of information related to inhalation performed by the user to a smartphone to cause the smartphone to display the items of information related to inhalation performed by the user. In another example, the communicator 115 receives information of a new OS from a server to update the information of the OS stored in the memory 114.

The controller 116 functions as an arithmetic processing unit and a control circuit, and controls the overall operations of the inhaler device 100 in accordance with various programs. The controller 116 is implemented by an electronic circuit such as a central processing unit (CPU) or a microprocessor, for example. In addition, the controller 116 may include a read-only memory (ROM) that stores a program to be used, an arithmetic parameter, and the like, and a random access memory (RAM) that temporarily stores a parameter that changes as appropriate and the like. The inhaler device 100 performs various processes under the control of the controller 116. Electric power supply from the power supply 111 to each of the other structural elements, charging of the power supply 111, detection of information by the sensor 112, notification of information by the notifier 113, storage and reading of information to and from the memory 114, and transmission and reception of information by the communicator 115 are an example of the processes controlled by the controller 116. Other processes performed by the inhaler device 100, such as input of information to each structural element and a process based on information output from each structural element are also controlled by the controller 116.

The holder 140 has an internal space 141, and holds the stick substrate 150 in a manner such that the stick substrate 150 is partially accommodated in the internal space 141. The holder 140 has an opening 142 that allows the internal space 141 to communicate with outside. The holder 140 holds the stick substrate 150 that is inserted into the internal space 141 through the opening 142. For example, the holder 140 may be a tubular body having the opening 142 and a bottom 143 that is a bottom surface, and may define the pillar-shaped internal space 141. The holder 140 has, in at least a portion of the tubular body in the height direction, an inside diameter that is smaller than an outside diameter of the stick substrate 150 to be able to hold the stick substrate 150 by pressing the stick substrate 150 inserted into the internal space 141 from the outer circumference. The holder 140 also has a function of defining a flow path of air that passes through the stick substrate 150. For example, the bottom 143 has an air inlet hole that is an inlet of air into the flow path. On the other hand, the opening 142 serves as an air outlet hole that is an outlet of air from the flow path.

The stick substrate 150 is a stick-shaped member. The stick substrate 150 includes a substrate 151 and an inhalation port 152.

The substrate 151 includes an aerosol source. The aerosol source is heated to be atomized, so that an aerosol is generated. The aerosol source may be a material derived from tobacco, such as shredded tobacco or a processed material obtained by forming a tobacco raw material into a granular, sheet-like, or powdery shape. In addition, the aerosol source may include a material that is not derived from tobacco, such as a material made from a plant other than tobacco (for example, mint or an herb). In an example, the aerosol source may include a flavor component such as menthol. For the inhaler device 100 that is a medical inhaler, the aerosol source may include a medicine to be inhaled by a patient. The aerosol source is not limited to a solid and may be a liquid such as polyhydric alcohol and water. Examples of the polyhydric alcohol include glycerine and propylene glycol. At least a portion of the substrate 151 is accommodated in the internal space 141 of the holder 140 when the stick substrate 150 is held by the holder 140

The inhalation port 152 is to be held in a mouth of the user during inhalation. At least a portion of the inhalation port 152 protrudes from the opening 142 when the stick substrate 150 is held by the holder 140. When a user performs inhalation while holding, in their mouth, the inhalation port 152 protruding from the opening 142, air flows into the holder 140 through the air inlet hole (not illustrated). The air that has flowed in passes through the internal space 141 of the holder 140, that is, the substrate 151, and reaches the inside of the mouth of the user together with the aerosol generated from the substrate 151.

The stick substrate 150 further includes the susceptor 161. The susceptor 161 produces heat by electromagnetic induction. The susceptor 161 may be made of a conductive material such as metal. In an example, the susceptor 161 is a piece of metal. The susceptor 161 is disposed in proximity to the aerosol source. In the example illustrated in Fig. 1, the susceptor 161 is included in the substrate 151 of the stick substrate 150.

The susceptor 161 is disposed in thermal proximity to the aerosol source. The susceptor 161 being in thermal proximity to the aerosol source means that the susceptor 161 is disposed at a position where heat produced by the susceptor 161 is transferred to the aerosol source. For example, the susceptor 161 is included in the substrate 151 along with the aerosol source and is surrounded by the aerosol source. This configuration enables the heat produced by the susceptor 161 to be efficiently used for heating the aerosol source.

Note that, the susceptor 161 may be untouchable from outside of the stick substrate 150. For example, the susceptor 161 may be distributed in a central part of the stick substrate 150, but does not have to be distributed near the outer circumference of the stick substrate 150.

The electromagnetic induction source 162 causes the susceptor 161 to produce heat by electromagnetic induction. For example, the electromagnetic induction source 162 is a coiled conductive wire wound around the outer circumference of the holder 140. Upon being supplied with an alternating current from the power supply 111, the electromagnetic induction source 162 generates a magnetic field. The electromagnetic induction source 162 is disposed at a position where the internal space 141 of the holder 140 overlaps with the generated magnetic field. Thus, when a magnetic field is generated while the stick substrate 150 is held by the holder 140, an eddy current is generated in the susceptor 161 to generate Joule heat. The aerosol source included in the stick substrate 150 is heated by the Joule heat to be atomized, so that an aerosol is generated. In an example, when the sensor 112 detects a predetermined user input, electric power may be supplied and an aerosol may be generated. When the temperature of the stick substrate 150 that is heated by induction heating using the susceptor 161 and the electromagnetic induction source 162 reaches a predetermined temperature, the user can perform inhalation. When the sensor 112 detects a predetermined user input thereafter, electric power supply may be stopped. In another example, electric power may be supplied and an aerosol may be generated, while the sensor 112 detects inhalation performed by the user

The combination of the inhaler device 100 and the stick substrate 150 may be regarded as a single system because an aerosol can be generated by combining the inhaler device 100 and the stick substrate 150.

### <2. Induction heating>

Induction heating will be described in detail below.

Induction heating is a process of heating a conductive object by causing a varying magnetic field to penetrate the object. Induction heating involves a magnetic field generator that generates a varying magnetic field, and a to-be-heated object that is conductive and is to be heated when exposed to the varying magnetic field. An example of the varying magnetic field is an alternating magnetic field. The electromagnetic induction source 162 illustrated in Fig. 1 is an example of the magnetic field generator. The susceptor 161 illustrated in Fig. 1 is an example of the to-be-heated object.

The magnetic field generator and the to-be-heated object are disposed at relative positions such that a varying magnetic field generated from the magnetic field generator penetrates the to-be-heated object. When a varying magnetic field is generated from the magnetic field generator in this state, an eddy current is induced in the to-be-heated object. The eddy current flows through the to-be-heated object, which produces Joule heat according to the electrical resistance of the to-be-heated object, so that the to-be-heated object is heated. Such heating is also referred to as Joule heating, ohmic heating, or resistive heating.

The to-be-heated object may be magnetic. In this case, the to-be-heated object is further heated by magnetic hysteresis heating. Magnetic hysteresis heating is a process of heating a magnetic object by causing a varying magnetic field to penetrate the object. When a magnetic field penetrates a magnetic body, magnetic dipoles included in the magnetic body are aligned along the magnetic field. Thus, when a varying magnetic field penetrates a magnetic body, the orientation of the magnetic dipoles changes in accordance with the applied varying magnetic field. Such reorientation of the magnetic dipoles produces heat in the magnetic body, so that the to-be-heated object is heated.

Magnetic hysteresis heating typically occurs at a temperature of the Curie point or lower and does not occur at a temperature exceeding the Curie point. The Curie point is the temperature at which a magnetic body loses magnetic properties thereof. For example, when the temperature of a to-be-heated object that is ferromagnetic at a temperature of the Curie point or lower exceeds the Curie point, a reversible phase transition from ferromagnetism to paramagnetism occurs in the magnetism of the to-be-heated object. When the temperature of the to-be-heated object exceeds the Curie point, magnetic hysteresis heating no longer occurs. Thus, the temperature increase rate slows down.

The to-be-heated object is desirably made of a conductive material. Further, the to-be-heated object is desirably made of a ferromagnetic material. This is because the combination of resistive heating and magnetic hysteresis heating can increase the heating efficiency in the latter case. For example, the to-be-heated object may be made of one or more materials selected from a material group including aluminum, iron, nickel, cobalt, conductive carbon, copper, and stainless steel.

In both resistive heating and magnetic hysteresis heating, heat is produced inside the to-be-heated object rather than by thermal conduction from an external heat source. This thus can implement a rapid temperature increase and a uniform heat distribution in the to-be-heated object. This can be implemented by appropriately designing the material and shape of the to-be-heated object and the magnitude and direction of the varying magnetic field. That is, a rapid temperature increase and a uniform heat distribution can be implemented in the stick substrate 150 by appropriately designing the distribution of the susceptor 161 included in the stick substrate 150. This thus can reduce the time for preheating and improve the quality of a flavor tasted by the user

Since induction heating directly heats the susceptor 161 included in the stick substrate 150, the substrate can be heated more efficiently than when the stick substrate 150 is heated from the outer circumference or the like by an external heat source. When heating is performed using an external heat source, the temperature of the external heat source inevitably becomes higher than that of the stick substrate 150. In contrast, when induction heating is performed, the temperature of the electromagnetic induction source 162 does not become higher than that of the stick substrate 150. Thus, the temperature of the inhaler device 100 can be maintained to be lower than that in the case of using an external heat source. This is a great advantage in terms of user safety.

The electromagnetic induction source 162 generates a varying magnetic field by using electric power supplied from the power supply 111. In an example, the power supply 111 may be a direct current (DC) power supply. In this case, the power supply 111 supplies, via a DC/alternate current (AC) inverter, AC electric power to the electromagnetic induction source 162. In this case, the electromagnetic induction source 162 can generate an alternating magnetic field.

The electromagnetic induction source 162 generates a varying magnetic field in the internal space 141 of the holder 140. Thus, the varying magnetic field generated from the electromagnetic induction source 162 penetrates the susceptor 161 disposed in thermal proximity to the aerosol source included in the stick substrate 150 held by the holder 140. The susceptor 161 produces heat upon being penetrated by the varying magnetic field. The electromagnetic induction source 162 illustrated in Fig. 1 is a solenoid coil. The solenoid coil is disposed such that the conductive wire is wound around the outer circumference of the holder 140. When a current is applied to the solenoid coil, a magnetic field is generated in a central space surrounded by the coil, that is, the internal space 141 of the holder 140. As illustrated in Fig. 1, the susceptor 161 is surrounded by the coil when the stick substrate 150 is held by the holder 140. Thus, the varying magnetic field generated from the electromagnetic induction source 162 penetrates the susceptor 161 and heats the susceptor 161 by induction heating.

### <3. Technical features>

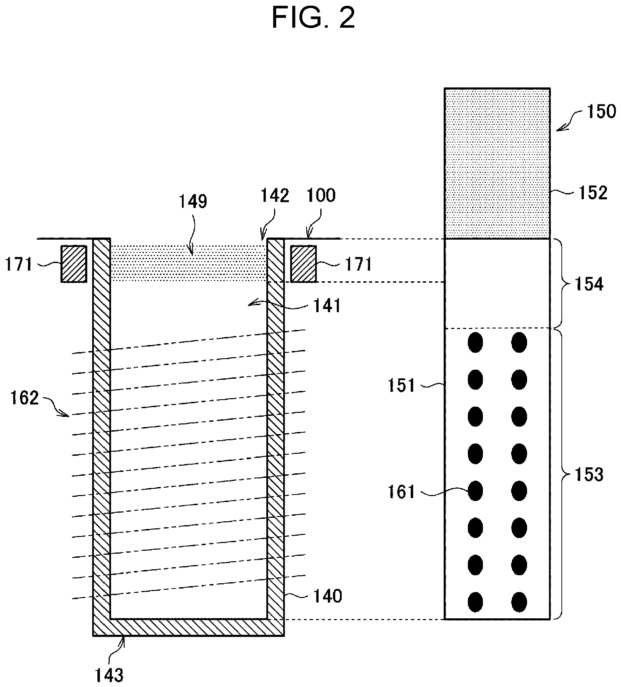

Fig. 2 is a diagram schematically illustrating a configuration around the holder 140 of the inhaler device 100 according to the present embodiment and a configuration of the stick substrate 150. Fig. 2 schematically illustrates a state in which a tip of the stick substrate 150 and the bottom 143 of the holder 140 are aligned at the same position with longitudinal directions of the stick substrate 150 and the holder 140 are kept parallel to each other. In the example illustrated in Fig. 2, when the stick substrate 150 is held by the holder 140, the tip of the stick substrate 150 is in contact with the bottom 143, the substrate 151 is accommodated in the holder 140, and the inhalation port 152 protrudes outward through the opening 142.

As illustrated in Fig. 2, the inhaler device 100 includes a detector 171. The detector 171 is an example of the sensor 112. The detector 171 detects information of a partial space 149 which is a portion of the internal space 141 of the holder 140 adjacent to the opening 142. With such a configuration, when the stick substrate 150 is inserted into the internal space 141 even halfway, a change occurs in the information of the partial space 149 detected by the detector 171. By contrast, for example, when the detector 171 is provided adjacently to the bottom 143, the change does not occur in the information detected by the detector 171 unless the stick substrate 150 is fully inserted. However, the above configuration enables the determination as to whether the stick substrate 150 is present to be performed even in a use case where the stick substrate 150 is not fully inserted.

The electromagnetic induction source 162 is disposed outside the internal space 141. More specifically, the electromagnetic induction source 162 is wound around the outer circumference of the holder 140 while avoiding a region near the opening 142. The detector 171 is disposed outside the internal space 141 to be closer to the opening 142 than the electromagnetic induction source 162 is. More specifically, the detector 171 is disposed adjacently to the opening 142 where the electromagnetic induction source 162 is not disposed and in proximity to the outer circumference of the holder 140. With such a configuration, once the tip of the stick substrate 150 is inserted into the internal space 141, the change occurs in the information detected by the detector 171. Thus, the determination as to whether the stick substrate 150 is present can be performed in various use cases. In such a configuration, the detector 171 is not disposed in a space surrounded by at least the electromagnetic induction source 162. This thus can prevent hindrance of induction heating by the presence of the detector 171.

The detector 171 is a non-contact proximity sensor. With such a configuration, the detector 171 can detect the information of the partial space 149 without hindering passage of the stick substrate 150 through the partial space 149.

The detector 171 may be a capacitive proximity sensor. The capacitive proximity sensor is a sensor that generates an electric field and detects a change in capacitance caused by entry of a target object to the electric filed to detect the target object. In this case, the detector 171 detects a capacitance, a dielectric constant, or the like as the information of the partial space 149. With such a configuration, information corresponding to a material of an object that is present in the partial space 149 can be detected and used for determining whether the stick substrate 150 is present.

The controller 116 determines whether the stick substrate 150 including the susceptor 161 is held by the holder 140, based on the information of the partial space 149 detected by the detector 171. With such a configuration, whether the stick substrate 150 is present can be determined without generating a varying magnetic field from the electromagnetic induction source 162. Thus, even when the stick substrate 150 is not held by the holder 140, the occurrence of a leakage flux can be prevented.

Further, when the controller 116 determines that the stick substrate 150 including the susceptor 161 is held by the holder 140, the controller 116 permits induction heating performed by the electromagnetic induction source 162. On the other hand, when the controller 116 determines that the stick substrate 150 including the susceptor 161 is not held by the holder 140, the controller 116 prohibits induction heating performed by the electromagnetic induction source 162. Such a configuration can prevent so-called empty heating in which induction heating is performed even though the stick substrate 150 including the susceptor 161 is not held by the holder 140. When empty heating is performed, a large leakage flux may occur. However, with the above configuration, the occurrence of the large leakage flux can be prevented.

Further, the controller 116 may start induction heating performed by the electromagnetic induction source 162 when determining that the stick substrate 150 including the susceptor 161 is held by the holder 140. More specifically, the controller 116 may start induction heating performed by the electromagnetic induction source 162 in response to a trigger that is a change from a state in which the stick substrate 150 is not held by the holder 140 to a state in which the stick substrate 150 is held by the holder 140. Such a configuration can improve convenience because induction heating is automatically started when the user inserts the stick substrate 150 into the inhaler device 100. In addition, when the stick substrate 150 is removed from the holder 140 during induction heating, the controller 116 may prohibit (that is, stop) induction heating. Such a configuration can prevent empty heating even when the stick substrate 150 unintentionally falls off during heating.

As illustrated in Fig. 2, the stick substrate 150 includes a first portion 153 where the susceptor 161 is distributed and a second portion 154 where the susceptor 161 is not distributed. When the stick substrate 150 is held by the holder 140, the first portion 153 is located closer to the bottom 143 than to the partial space 149. In addition, when the stick substrate 150 is held by the holder 140, the second portion 154 is located in the partial space 149. With such a configuration, the information detected by the detector 171 is different between the state in which the stick substrate 150 is inserted halfway and the state in which the stick substrate 150 is fully inserted. This thus enables whether the stick substrate 150 is present to be accurately determined.

The controller 116 determines whether the stick substrate 150 including the susceptor 161 is held by the holder 140, based on a time-series change in the information of the partial space 149. As the stick substrate 150 is inserted into the internal space 141 of the holder 140 or removed from the internal space 141, the portion of the stick substrate 150 located in the partial space 149 changes. Accordingly, the information of the partial space 149 detected by the detector 171 changes. Thus, whether the stick substrate 150 is present can be determined by using the fact that the information of the partial space 149 changes in response to a user operation of inserting or removing the stick substrate 150.

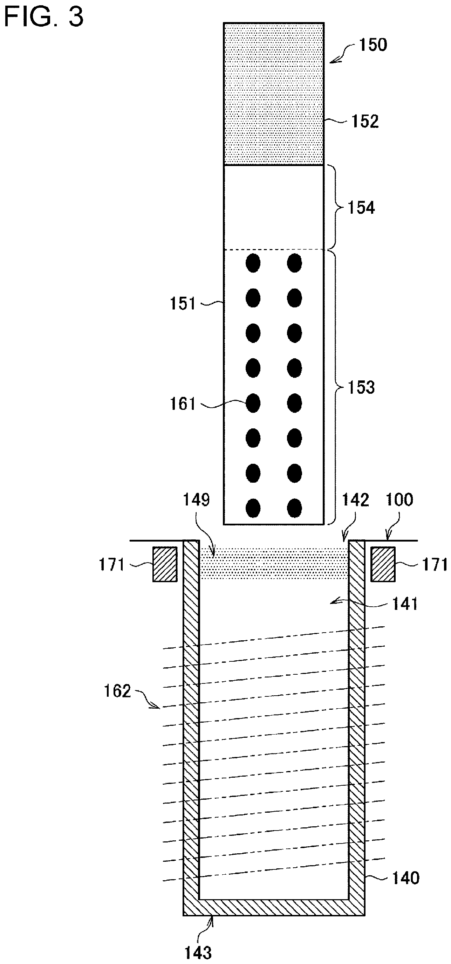

The controller 116 determines which of first to third states the state of the partial space 149 is, based on the information of the partial space 149 detected by the detector 171. In an example, the controller 116 determines that the state of the partial space 149 is the first state if the detected capacitance or dielectric constant is within a first range, determines that the state of the partial space 149 is the second state if the detected capacitance or dielectric constant is within a second range, and determines that the state of the partial space 149 is the third state if the detected capacitance or dielectric constant is within a third range. The controller 116 then determines whether the stick substrate 150 including the susceptor 161 is held by the holder 140, based on the time-series change in the state of the partial space 149. A process of determining whether the stick substrate 150 is present will be described below with reference to Figs. 3 to 5.

Fig. 3 is a diagram schematically illustrating a positional relationship between the holder 140 and the stick substrate 150 in the first state. As illustrated in Fig. 3, the first state is a state in which an object is not present in the partial space 149. Accordingly, a state in which even the tip of the stick substrate 150 is not inserted into the holder 140 is determined to be the first state.

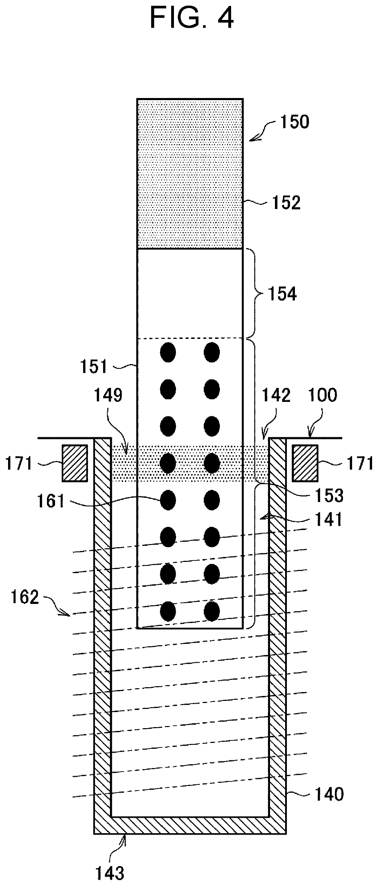

Fig. 4 is a diagram schematically illustrating a positional relationship between the holder 140 and the stick substrate 150 in the second state. As illustrated in Fig. 4, the second state is a state in which an object including a conductor is present in the partial space 149. The second state also includes a state in which a conductor and a non-conductor are present in the partial space 149. Accordingly, a state in which the stick substrate 150 is inserted into the holder 140 halfway and the first portion 153 is located in the partial space 149 is determined to be the second state.

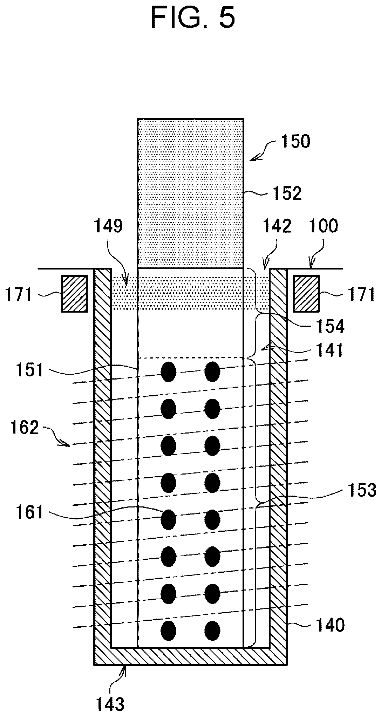

Fig. 5 is a diagram schematically illustrating a positional relationship between the holder 140 and the stick substrate 150 in the third state. As illustrated in Fig. 5, the third state is a state in which an object not including a conductor is present in the partial space 149. That is, the third state is a state in which an object including a non-conductor alone is present in the partial space 149. Accordingly, a state in which the stick substrate 150 is inserted fully such that the tip of the stick substrate 150 is in contact with the bottom 143 of the holder 140 and the second portion 154 is located in the partial space 149 is determined to be the third state.

In an example, when the state of the partial space 149 changes from the first state to the second state and then to the third state, the controller 116 determines that the stick substrate 150 including the susceptor 161 is held by the holder 140. With such a configuration, it can be determined that the stick substrate 150 is fully inserted by using the fact that the state of the partial space 149 changes in response to the user operation of inserting the stick substrate 150.

When the state of the partial space 149 changes from the first state to the second state and then to the third state, the controller 116 may determine that the stick substrate 150 including the susceptor 161 is held by the holder 140 and also identify the stick substrate 150. In this case, the controller 116 identifies the stick substrate 150 held by the holder 140, based on the time-series change in the information of the partial space 149. The shape and distribution of the susceptor 161 included in the stick substrate 150 may vary depending on the type of the stick substrate 150. The time-series change in the information of the partial space 149, such as the capacitance and the dielectric constant, which occur when the first portion 153 passes through the partial space 149, may vary depending on the shape and distribution of the susceptor 161. Thus, the type of the stick substrate 150 is identifiable based on the time-series change in the information of the partial space 149 in the state in which the first portion 153 is passing through the partial space 149, that is, in the second state. With such a configuration, not only the presence or absence of the stick substrate 150 but also the stick substrate 150 can be identified.

In another example, when the state of the partial space 149 changes from the third state to the second state and then to the first state, the controller 116 determines that the stick substrate 150 including the susceptor 161 that has been held by the holder 140 is removed. With such a configuration, it can be determined that the stick substrate 150 is removed by using the fact that the state of the partial space 149 changes in response to the user operation of removing the stick substrate 150.

In another example, when the state of the partial space 149 does not change from the first state, the controller 116 determines that the stick substrate 150 including the susceptor 161 is not held by the holder 140. With such a configuration, it can be determined that even the tip of the stick substrate 150 is not inserted.

In another example, when the state of the partial space 149 changes from the first state to the second state and then to the first state, the controller 116 determines that the stick substrate 150 including the susceptor 161 is inserted halfway and then removed. With such a configuration, it can be determined that the stick substrate 150 is inserted halfway and then removed by using the fact that the state of the partial space 149 changes in response to the user operation of inserting the stick substrate 150 halfway and then removing the stick substrate 150.

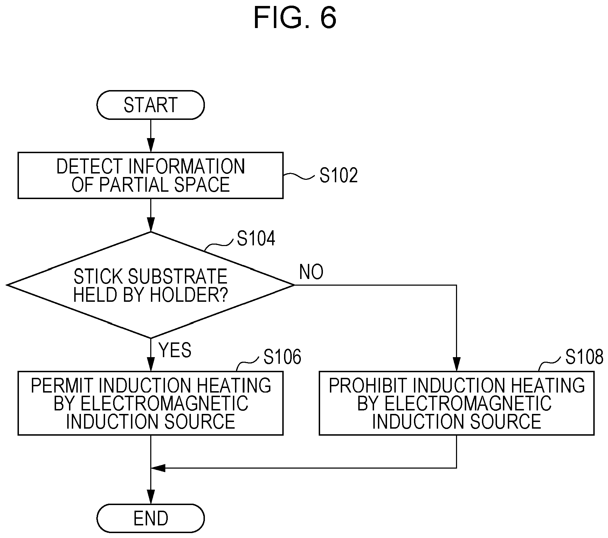

A procedure of a process of determining whether the stick substrate 150 is present will be described below with reference to Fig. 6. Fig. 6 is a flowchart illustrating an example of the procedure of the process of determining whether the stick substrate 150 is present, performed by the inhaler device 100 according to the present embodiment.

As illustrated in Fig. 6, the detector 171 detects the information of the partial space 149 (step S102).

Then, the controller 116 determines whether the stick substrate 150 is held by the holder 140, based on the time-series change in the information of the partial space 149 (step S104). For example, when the state of the partial space 149 changes from the first state to the second state and then to the third state, the controller 116 determines that the stick substrate 150 including the susceptor 161 is held by the holder 140. Otherwise, the controller 116 determines that the stick substrate 150 including the susceptor 161 is not held by the holder 140.

If determining that the stick substrate 150 is held by the holder 140 (step S104: YES), the controller 116 permits induction heating performed by the electromagnetic induction source 162 (step S106). Thus, in response to a user operation for a heating start instruction, induction heating is started and the user can perform a puff. The process then ends.

On the other hand, if determining that the stick substrate 150 is not held by the holder 140 (step S104: NO), the controller 116 prohibits induction heating performed by the electromagnetic induction source 162 (step S108). Thus, even if a user operation for a heating start instruction is performed, induction heating is not started and thus empty heating can be prevented. The process then ends.

### <4. Supplementary description>

While the preferred embodiment of the present invention has been described in detail above with reference to the accompanying drawings, the present invention is not limited to such examples. Obviously, a person with an ordinary knowledge in the technical field to which the present invention pertains can conceive various modifications and corrections within the scope of the technical spirit described in the claims. It should be understood that these modifications and corrections naturally pertain to the technical scope of the present invention.

For example, in the embodiment above, an example has been described in which the detector 171 is a capacitive proximity sensor. However, the present invention is not limited to such an example. For example, the detector 171 may be an inductive proximity sensor. The inductive proximity sensor is a sensor that generates a varying magnetic field and detects an approach of a conductor by using the fact that an induced current caused by electromagnetic induction flows through the conductor in response to the approach of the conductor to cause a heat loss. In this case, the detector 171 detects an impedance or the like of a circuit that generates the varying magnetic field, as the information of the partial space 149. With such a configuration, whether the stick substrate 150 is held by the holder 140 can be determined without generating the varying magnetic field at least from the electromagnetic induction source 162. Typically, a magnetic field generated from the inductive proximity sensor is weaker than the magnetic field generated from the electromagnetic induction source 162. Thus, an influence of the leakage flux can be reduced as compared with an example in which whether the stick substrate 150 is present is determined using the magnetic field generated from the electromagnetic induction source 162. Alternatively, the detector 171 may be a photoelectric proximity sensor. The photoelectric proximity sensor is a proximity sensor that detects an object that blocks an optical axis between a light-projecting unit and a light-receiving unit disposed to face each other.

In addition, the detector 171 may include an inductive proximity sensor and a photoelectric proximity sensor. In this case, the first state to the third state may be determined based on a combination of detection results. For example, when the photoelectric proximity sensor does not detect any object, the controller 116 determines that the stick substrate 150 is not inserted, that is, the state of the partial space 149 is the first state. When the photoelectric proximity sensor detects an object and the inductive proximity sensor detects a conductor, the controller 116 determines that the stick substrate 150 is inserted halfway, that is, the state of the partial space 149 is the second state. When the photoelectric proximity sensor detects an object and the inductive proximity sensor does not detect a conductor, the controller 116 determines that the stick substrate 150 is fully inserted, that is, the state of the partial space 149 is the third state.

For example, in the embodiment described above, an example has been described in which the susceptor 161 is distributed to the tip of the stick substrate 150. However, the present invention is not limited to such an example. For example, the susceptor 161 is not distributed at the tip of the stick substrate 150, that is, the second portion 154 may be present also at the tip of the stick substrate 150. In this case, when the state of the partial space 149 changes from the first state to the third state, then to the second state, and lastly to the third state, the controller 116 determines that the stick substrate 150 including the susceptor 161 is held by the holder 140. As described above, an algorithm for determining whether the stick substrate 150 is present may be appropriately designed in accordance with distribution of the susceptor 161 in the stick substrate 150.

For example, in the embodiment above, an example has been described in which the susceptor 161 is a piece of metal. However, the present invention is not limited to such an example. For example, the susceptor 161 may have an elongated shape such as a rod shape, a cylindrical shape, or a plate shape. In this case, the susceptor 161 is desirably disposed at the center of the substrate 151 to extend in a longitudinal direction of the substrate 151. With such a configuration, an aerosol can be generated in a short time from the start of heating since the susceptor 161 that produces a large amount of heat by induction heating is disposed at the center of the substrate 151. Obviously, the susceptors 161 having a plurality of shapes may coexist in the substrate 151.

The series of steps performed by the individual devices described in this specification may be implemented by using any of software, hardware, and a combination of software and hardware. Programs constituting software are, for example, stored in advance in recording media (non-transitory media) provided inside or outside the individual devices. Each program is, for example, at the time of being executed by a computer that controls each of the devices described in this specification, loaded into a RAM and executed by a processor such as a CPU. The recording media are, for example, a magnetic disk, an optical disc, a magneto-optical disk, a flash memory, and the like. The computer programs may be distributed, for example, via a network without using recording media.

The steps described using a flowchart and a sequence diagram in this specification need not necessarily be executed in the order illustrated. Some of the process steps may be executed in parallel. An additional process step may be adopted, or one or some of the process steps may be omitted.

Configurations below also pertain to the technical scope of the present invention.
(1) An inhaler device including:
   a power supply configured to supply electric power;
   a holder having an internal space and an opening that allows the internal space to communicate with outside and configured to hold a substrate inserted into the internal space through the opening, the substrate including an aerosol source and a susceptor configured to produce heat upon being penetrated by a varying magnetic field;
   an electromagnetic induction source configured to generate the varying magnetic field in the internal space of the holder by using the electric power supplied from the power supply;
   a detector configured to detect information of a partial space that is a portion of the internal space adjacent to the opening; and
   a controller configured to determine whether the substrate is held by the holder, based on the information of the partial space detected by the detector.
(2) The inhaler device according to (1), in which
   the controller is configured to determine whether the substrate including the susceptor is held by the holder, based on a time-series change in the information of the partial space.
(3) The inhaler device according to (2), in which
   the controller is configured to: determine which of a first state, a second state, or a third state a state of the partial space is, based on the information of the partial space detected by the detector, the first state being a state in which an object is not present in the partial space, the second state being a state in which an object including a conductor is present in the partial space, the third state being a state in which an object not including a conductor is present in the partial space; and determine whether the substrate including the susceptor is held by the holder, based on a time-series change in the state of the partial space.
(4) The inhaler device according to (3), in which
   the controller is configured to determine that the substrate including the susceptor is held by the holder in a case where the state of the partial space changes from the first state to the second state and then to the third state.
(5) The inhaler device according to (3) or (4), in which
   the controller is configured to determine that the substrate including the susceptor that has been held by the holder is removed in a case where the state of the partial space changes from the third state to the second state and then to the first state.
(6) The inhaler device according to any one of (3) to (5), in which
   the controller is configured to determine that the substrate including the susceptor is not held by the holder in a case where the state of the partial space does not change from the first state.
(7) The inhaler device according to any one of (3) to (6), in which
   the controller is configured to determine that the substrate including the susceptor is inserted halfway and then removed in a case where the state of the partial space changes from the first state to the second state and then to the first state.
(8) The inhaler device according to any one of (2) to (7), in which
   the controller is configured to identify the substrate held by the holder, based on the time-series change in the information of the partial space.
(9) The inhaler device according to any one of (1) to (8), in which
   the controller is configured to start induction heating using the electromagnetic induction source in response to determining that the substrate including the susceptor is held by the holder.
(10) The inhaler device according to any one of (1) to (9), in which
   the substrate includes a first portion where the susceptor is distributed and a second portion where the susceptor is not distributed, and
   the second portion is located in the partial space when the substrate is held by the holder.
(11) The inhaler device according to any one of (1) to (10), in which
   the electromagnetic induction source is disposed outside the internal space, and
   the detector is disposed outside the internal space to be closer to the opening than the electromagnetic induction source is.
(12) The inhaler device according to any one of (1) to (11), in which
   the detector is a non-contact proximity sensor
(13) The inhaler device according to (12), in which
   the detector is a capacitive proximity sensor
(14) The inhaler device according to (12), in which
   the detector is an inductive proximity sensor.
(15) A program to be executed by a computer that controls an inhaler device,
   the inhaler device including:
      a power supply configured to supply electric power;
      a holder having an internal space and an opening that allows the internal space to communicate with outside and configured to hold a substrate inserted into the internal space through the opening, the substrate including an aerosol source and a susceptor configured to produce heat upon being penetrated by a varying magnetic field;
      an electromagnetic induction source configured to generate the varying magnetic field in the internal space of the holder by using the electric power supplied from the power supply; and
      a detector configured to detect information of a partial space that is a portion of the internal space adjacent to the opening,
   the program causing
      determining whether the substrate is held by the holder, based on the information of the partial space detected by the detector
   to be performed.
(16) A system including: an inhaler device; and a substrate,
   the substrate including an aerosol source and a susceptor configured to produce heat upon being penetrated by a varying magnetic field,
   the inhaler device including:
      a power supply configured to supply electric power;
      a holder having an internal space and an opening that allows the internal space to communicate with outside and configured to hold the substrate inserted into the internal space through the opening;
      an electromagnetic induction source configured to generate the varying magnetic field in the internal space of the holder by using the electric power supplied from the power supply;
      a detector configured to detect information of a partial space that is a portion of the internal space adjacent to the opening; and
      a controller configured to determine whether the substrate is held by the holder, based on the information of the partial space detected by the detector.

### Reference Signs List

- 100: inhaler device
- 111: power supply
- 112: sensor
- 113: notifier
- 114: memory
- 115: communicator
- 116: controller
- 121: heater
- 140: holder
- 141: internal space
- 142: opening
- 143: bottom
- 144: heat insulator
- 149: partial space
- 150: stick substrate
- 151: substrate
- 152: inhalation port
- 153: first portion
- 154: second portion
- 161: susceptor
- 162: electromagnetic induction source
- 171: detector

## Claims

1. An inhaler device comprising:
a power supply configured to supply electric power;
a holder having an internal space and an opening that allows the internal space to communicate with outside and configured to hold a substrate inserted into the internal space through the opening, the substrate including an aerosol source and a susceptor configured to produce heat upon being penetrated by a varying magnetic field;
an electromagnetic induction source configured to generate the varying magnetic field in the internal space of the holder by using the electric power supplied from the power supply;
a detector configured to detect information of a partial space that is a portion of the internal space adjacent to the opening; and
a controller configured to determine whether the substrate is held by the holder, based on the information of the partial space detected by the detector.

2. The inhaler device according to claim 1, wherein
the controller is configured to determine whether the substrate including the susceptor is held by the holder, based on a time-series change in the information of the partial space.

3. The inhaler device according to claim 2, wherein
the controller is configured to: determine which of a first state, a second state, or a third state a state of the partial space is, based on the information of the partial space detected by the detector, the first state being a state in which an object is not present in the partial space, the second state being a state in which an object including a conductor is present in the partial space, the third state being a state in which an object not including a conductor is present in the partial space; and determine whether the substrate including the susceptor is held by the holder, based on a time-series change in the state of the partial space.

4. The inhaler device according to claim 3, wherein
the controller is configured to determine that the substrate including the susceptor is held by the holder in a case where the state of the partial space changes from the first state to the second state and then to the third state.

5. The inhaler device according to claim 3 or 4, wherein
the controller is configured to determine that the substrate including the susceptor that has been held by the holder is removed in a case where the state of the partial space changes from the third state to the second state and then to the first state.

6. The inhaler device according to any one of claims 3 to 5, wherein
the controller is configured to determine that the substrate including the susceptor is not held by the holder in a case where the state of the partial space does not change from the first state.

7. The inhaler device according to any one of claims 3 to 6, wherein
the controller is configured to determine that the substrate including the susceptor is inserted halfway and then removed in a case where the state of the partial space changes from the first state to the second state and then to the first state.

8. The inhaler device according to any one of claims 2 to 7, wherein
the controller is configured to identify the substrate held by the holder, based on the time-series change in the information of the partial space.

9. The inhaler device according to any one of claims 1 to 8, wherein
the controller is configured to start induction heating using the electromagnetic induction source in response to determining that the substrate including the susceptor is held by the holder.

10. The inhaler device according to any one of claims 1 to 9, wherein
the substrate includes a first portion where the susceptor is distributed and a second portion where the susceptor is not distributed, and
the second portion is located in the partial space when the substrate is held by the holder.

11. The inhaler device according to any one of claims 1 to 10, wherein
the electromagnetic induction source is disposed outside the internal space, and
the detector is disposed outside the internal space to be closer to the opening than the electromagnetic induction source is.

12. The inhaler device according to any one of claims 1 to 11, wherein
the detector is a non-contact proximity sensor.

13. The inhaler device according to claim 12, wherein
the detector is a capacitive proximity sensor.

14. The inhaler device according to claim 12, wherein
the detector is an inductive proximity sensor.

15. A program to be executed by a computer that controls an inhaler device,
the inhaler device including:
a power supply configured to supply electric power;
a holder having an internal space and an opening that allows the internal space to communicate with outside and configured to hold a substrate inserted into the internal space through the opening, the substrate including an aerosol source and a susceptor configured to produce heat upon being penetrated by a varying magnetic field;
an electromagnetic induction source configured to generate the varying magnetic field in the internal space of the holder by using the electric power supplied from the power supply; and
a detector configured to detect information of a partial space that is a portion of the internal space adjacent to the opening,
the program causing
determining whether the substrate is held by the holder, based on the information of the partial space detected by the detector
to be performed.

16. A system comprising: an inhaler device; and a substrate,
the substrate including an aerosol source and a susceptor configured to produce heat upon being penetrated by a varying magnetic field,
the inhaler device including:
a power supply configured to supply electric power;
a holder having an internal space and an opening that allows the internal space to communicate with outside and configured to hold the substrate inserted into the internal space through the opening;
an electromagnetic induction source configured to generate the varying magnetic field in the internal space of the holder by using the electric power supplied from the power supply;
a detector configured to detect information of a partial space that is a portion of the internal space adjacent to the opening; and
a controller configured to determine whether the substrate is held by the holder, based on the information of the partial space detected by the detector.
